# EUROPÄISCHE PATENTSCHRIFT

(11) **EP 2 501 781 B1**
(45) Veröffentlichungstag und Bekanntmachung des Hinweises auf die Patenterteilung: **19.03.2014**
(21) Anmeldenummer: 10776380.7
(22) Anmeldetag: 16.11.2010
(51) Int. Cl.: C10G 2/00, B01J 23/745, B01J 31/20

(54) **VERFAHREN ZUR SELEKTIVEN HERSTELLUNG VON LEICHTEN OLEFINEN**
PROCESS FOR SELECTIVELY PRODUCING LIGHT OLEFINS
PROCÉDÉ POUR LA PRODUCTION SÉLECTIVE D'OLÉFINES LÉGÈRES

(30) Priorität: 19.11.2009 EP 09176550
(43) Veröffentlichungstag der Anmeldung: 26.09.2012
(73) Patentinhaber: BASF SE, 67056 Ludwigshafen (DE)
(72) Erfinder: STEINER, Jochen, 64625 Bensheim (DE); BAY, Kerem, 67063 Ludwigshafen (DE); SCHWAB, Ekkehard, 67434 Neustadt (DE); WECK, Alexander, 67251 Freinsheim (DE)
(86) Internationale Anmeldenummer: PCT/EP2010/067567
(87) Internationale Veröffentlichungsnummer: WO 2011/061183

(56) Entgegenhaltungen:
- WO-A2-2006/134471
- WO-A2-2009/013174
- US-A1- 2005 113 465
- JANARDANARAO M: "Direct Catalytic Conversion of Synthesis Gas to Lower Olefins", INDUSTRIAL & ENGINEERING CHEMISTRY RESEARCH, [Online] Bd. 29, Nr. 9, September 1990 (1990-09), Seiten 1735-1753, XP002628374, DOI: 10.1021/ie00105a001 [gefunden am 2011-03-16]

## Beschreibung

Die Erfindung betrifft ein Verfahren zur selektiven Herstellung von Olefinen mittels Fischer-Tropsch-Synthese, insbesondere die selektive Herstellung von C₂-C₈-Olefinen. Darüber hinaus ist das Verfahren auch emissionsarm, weil das unter anderem bei der Fischer-Tropsch-Synthese als Nebenprodukt anfallende Kohlendioxid in das Verfahren rückgeleitet wird.

Lineare oder verzweigte Alkene, die 2 bis 8, bevorzugt 2 bis 6 und insbesondere 2 bis 4 Kohlenstoffatome besitzen, werden auch als niedrigere oder leichte Olefine bezeichnet. Es ist beispielsweise aus WO 2009/013174 bekannt, dass leichte Olefine aus Kohlenmonoxid (CO) und Wasserstoff (H₂) an Metallkatalysatoren, zum Beispiel Eisenoder Kobalt-Katalysatoren, hergestellt werden können.

Als Fischer-Tropsch-Synthese wird generell die Umsetzung von Kohlenmonoxid mit Wasserstoff in Gegenwart eines Heterogenkatalysators bezeichnet. Eine Mischung enthaltend im Wesentlichen Kohlenmonoxid und Wasserstoff - vorzugsweise hergestellt aus Erdgas durch Umsetzung mit Wasser (oder Sauerstoff) - wird auch Synthesegas genannt.

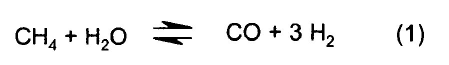

Mittels der Fischer-Tropsch-Synthese werden durch die Umsetzung der Komponenten des Synthesegases an einem geeigneten Heterogenenkatalysator Kohlenwasserstoffe, insbesondere Alkane und Olefine, hergestellt, wobei die Produktverteilung stark von den eingestellten Reaktionsparametern abhängt. Nachfolgend ist die chemische Reaktionsgleichung für die Olefinsynthese dargestellt.

2*n* H₂ + *n* CO - CₙH₂ₙ + *n*H₂O (2)

In Gegenwart eines eisenhaltigen Katalysatores wird in der Regel zusätzlicher Wasserstoff aus Wasser und Kohlenmonoxid gebildet, weshalb in einer Fischer-TropschSynthese häufig ein auf das Kohlenmonoxid bezogener Überschuss an Wasserstoff während des Prozesses vorliegt. Kohlenmonoxid und Wasserstoff stehen in Gleichgewicht miteinander, wobei auf Seiten des Wasserstoffs auch das Treibhausgas KohlenEK09-0991 PC dioxid (CO₂) gebildet wird (siehe Gleichung (3)) Darüber hinaus wird bei der Fischer-Tropsch-Synthese zusätzliches Kohlendioxid als Nebenprodukt gebildet.

CO + H₂O ⇄ H₂ + CO₂ (3)

In WO 2009/013174 A2 wird ein Verfahren zur Herstellung von Olefinen durch Umsetzung von Kohlenmonoxid mit Wasserstoff in Gegenwart eines eisenhaltigen Heterogenkatalysators, der im Wesentlichen ein Carbonyl-Eisenpulver-Katalysator mit spherischen Primärpartikeln umfasst, beschrieben.

In WO-A 2009/051353 wird ein Verfahren zur direkten Synthese von leichten Kohlenwasserstoffen aus Erdgas beschrieben. Unter Anwesenheit eines Nickel-Aluminium-Katalysators wird zunächst aus Erdgas in einem Steam-Reforming-Prozess Synthesegas mit einem molaren Verhältnis von Kohlenmonoxid und Wasserstoff von 1 : 1,5 bis 2,5 hergestellt. In dem sich anschließenden Fischer-Tropsch-Verfahren werden aus Kohlenmonoxid und Wasserstoff unter Anwesenheit eines Eisen-Kupfer-Kaliumzeolithe-Katalysators leichte gesättigte Kohlenwasserstoffe (C₂-C₄) sowie als Nebenprodukte Methan, Kohlendioxid und C₂-C₄-Olefine gebildet. Die Nebenprodukte Kohlendioxid und Methan werden anschließend dem Prozess wieder in den Steam-Reforming-Schritt zugeführt.

Ein Problem bei der Fischer-Tropsch-Synthese (zur Herstellung von Olefinen) stellt die unerwünschte Bildung (Emission) von Kohlendioxid dar, welches als Treibhausgas für die Umwelt eine schädigende Wirkung aufweist.

Der vorliegenden Erfindung liegt die Aufgabe zugrunde, ein Verfahren bereitzustellen, womit die emissionsarme und/oder selektive Herstellung von Olefinen, vorzugsweise leichten Olefinen, über einen Fischer-Tropsch-Syntheseprozess ermöglicht wird.

Erfindungsgemäß wird die Aufgabe gelöst durch ein Verfahren zur Herstellung von Olefinen umfassend die folgenden Schritte:
a) Herstellung eines Kohlenmonoxid und Wasserstoff enthaltenden Synthesegases, wobei das Synthesegas aus einem Gemisch enthaltend Kohlenwasserstoffe mit einem Verhältnis von Wasserstoff zu Kohlenstoff von 0.5 bis 4:1 hergestellt wird,
b) Einspeisung von aus Schritt d) rückgeleitetem Kohlendioxid in das Synthesegas während oder im Anschluss an die Synthesegasherstellung gemäß Schritt a),
c) Umsetzen des in Schritt b) erhaltenen Synthesegases mit einem Wasserstoff-zu-Kohlenmonoxid-Verhältnis von ≤ 1,2 : 1 zu Olefinen in Gegenwart eines FischerTropsch-Katalysators,
d) Abtrennen des im Reaktionsprodukt von Schritt c) enthaltenen Kohlendioxids,
wobei das Verhältnis von Wasserstoff zu Kohlenmonoxid in Schritt c) durch Schritt b) eingestellt wird.

Im erfindungsgemäßen Verfahren wird vorzugsweise nur das in Schritt d) abgetrennte Kohlendioxid rückgeleitet und in Schritt b) verwendet. Die Rückleitung enthält kein Methan oder nur Spuren von Methan.

Das Synthesegas gemäß Schritt a) kann nach allgemein bekannten Verfahren (wie zum Beispiel beschrieben in Weissermehl et al., Industrial Organic Chemistry, Whiley-VCH, Weinheim, 2003, Seiten 15 bis 24) hergestellt werden.

Für die Synthesegas-Herstellung in Schritt a) können alle dafür bekannten Prozesse verwendet werden. In einer Ausgestaltung der Erfindung wird für die SynthesegasHerstellung in Schritt a) ein Prozess verwendet, der ausgewählt ist aus der Gruppe Steam-Reforming, autothermales Reforming, nicht-katalytische partiale Oxidation, katalytische partiale Oxidation, Wärmeaustausch-Reforming, Kompakt-Reforming und Dry-Reforming.

Bevorzugt erfolgt die Synthesegas-Herstellung in Schritt a) über einen Steam-Reforming oder Dry-Reforming-Prozess.

In einer bevorzugten Ausgestaltung der Erfindung wird das Synthesegas aus Methan und/oder Erdgas hergestellt.

In Schritt a) wird das Synthesegas aus einem Gemisch enthaltend Kohlenwasserstoffe mit einem Verhältnis von Wasserstoff zu Kohlenstoff von 0,5 bis 4 : 1 hergestellt.

In einer weiteren Ausführungsform beträgt dieses Verhältnis 1,2 bis 5 : 1.

Für die Synthesegas-Herstellung gemäß Schritt a) können geeignete Übergangsmetallkatalysatoren verwendet. Diese werden allerdings auch häufig in Kombination mit Hauptgruppen-Metallen verwendet. Beispiele für Metalle, die Anwendung in der Synthesegas-Herstellung finden, sind Nickel und Aluminium.

Als Fischer-Tropsch-Katalysatoren können alle dem Fachmann bekannten Katalysatoren, die für Fischer-Tropsch-Synthesen gängig sind, verwendet werden. Vorzugsweise kann in Schritt c) ein Fischer-Tropsch-Katalysator verwendet werden, der ein eisenoder kobalthaltiger Heterogenkatalysator ist.

Besonders bevorzugt ist in Schritt c) ein Fischer-Tropsch-Katalysator, der ein Carbonyl-Eisenpulver-Katalysator mit spherischen Primärpartikeln ist. Solche Katalysatoren sind in WO 2009/013174 beschrieben.

Das erfindungsgemäße Verfahren kann in Gegenwart von Wasser oder gegebenenfalls anderen Lösungsmitteln durchgeführt werden. Geeignete Lösungsmittel sind dem Fachmann bekannt.

Das erfindungsgemäße Verfahren kann in allen dem Fachmann bekannten Reaktoren für Fischer-Tropsch-Synthesen mit der Möglichkeit der Gasrückleitung durchgeführt werden. In einer weiteren Ausgestaltung der Erfindung werden als Reaktoren zur Durchführung des erfindungsgemäßen Verfahrens Wirbelschichtreaktoren, Festbettreaktoren und Suspensionsreaktoren vorzugsweise mit Gasrückleitfunktion verwendet.

Der Einsatz solcher Reaktoren für die Fischer-Tropsch-Synthese ist zum Beispiel beschrieben in C. D. Frohning et al. in "Chemierohstoffe aus Kohle", 1977, Seiten 219 bis 299, oder B. H. Davis, Tropics and Catalysis, 2005, 32 (3 bis 4), Seiten 143 bis 168.

In einer besonderen Ausgestaltung der Erfindung liegen Wasserstoff zu Kohlenmonoxid in Schritt c) in einem Verhältnis von 1,2 : 1 bis 1 : 1,2 zueinander vor.

In einer weiteren Ausgestaltung der Erfindung liegen Wasserstoff zu Kohlenmonoxid in Schritt c) im Verhältnis 1,1 : 1 bis 1 : 1,1 liegt.

Besonders bevorzugt liegen Wasserstoff zu Kohlenmonoxid in Schritt c) im Verhältnis 1 : 1,1 zueinander vor.

In einer weiteren Ausführungsform liegen Wasserstoff zu Kohlenmonoxid in Schritt c) im Verhältnis 1 : 1 zueinander vor.

In dem erfindungsgemäßen Verfahren kann die Prozesstemperatur in Schritt c) 100 °C bis 500 °C betragen.

In einer weiteren Ausgestaltung der Erfindung wird Schritt c) bei Überdruck durchgeführt. Der Druck kann zwischen 5 und 50 bar betragen. Besonders bevorzugt beträgt der Druck in Schritt c) 20 bis 40 bar.

Das erfindungsgemäße Verfahren liefert ein Produktgemisch enthaltend Olefine, vorzugsweise leichte Olefine (C₂-C₈-Olefine), mehr bevorzugt C₂-C₆-Olefine, besonders bevorzugt C₂-C₄-Olefine, sowie geringe Mengen an aliphatischen, gesättigten Kohlenwasserstoffen mit mindestens 2 Kohlenstoffatomen. In dem erfindungsgemäßen Verfahren liegt die Ausbeute an Olefinen bei bis zu 47 Gew.-% bezogen auf die gesamte Kohlenstoffausbeute.

Der Begriff "C₂-C₈-Olefine" bedeutet, dass die jeweiligen Olefine mindestens 2, aber nicht mehr als 8 Kohlenstoffatome aufweisen. Sie können linear oder verzweigt sein, vorzugsweise sind sie linear. Geeignete C₂-C₈-Olefine umfassen somit Ethen, Propen, Buten, Penten, Hexen, Hepten und Octen.

Die erhaltenen Olefine werden zum Beispiel in Verfahren zur Herstellung von Polyolefinen, Epoxiden, Oxoprodukten, Acrylnitrilen, Acrolein, Styrol eingesetzt.

Nachfolgend wird die Erfindung durch die Figuren näher beschrieben.

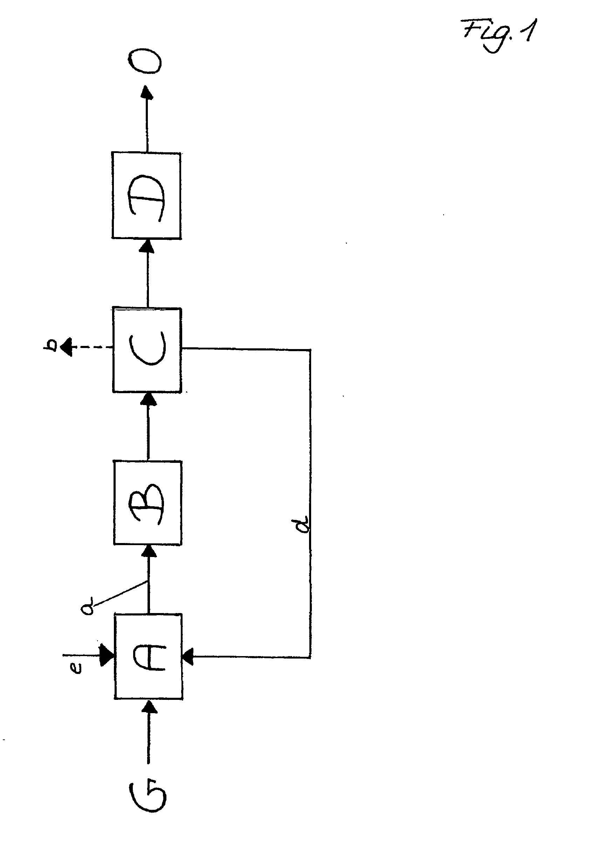

### Figur 1 beschreibt die Erfindung in der Ausgestaltung über den Dry-Reforming-Prozess

Das Erdgas (G), im Wesentlichen enthaltend Methan, wird in den Erdgas-Reformer (A) zur Herstellung des Synthesegases geleitet, wobei zusätzlich Energie (e) zugeführt wird. Durch während des Reaktionsprozesses abgetrenntes Kohlendioxid (d) und Rückführung in den Erdgas-Reformer (A) wird ein Synthesegas mit einem Verhältnis von Wasserstoff zu Kohlenmonoxid von vorzugsweise 1 : 1 (a) eingestellt. Dieses Synthesegas wird dann in den Fischer-Tropsch-Reaktor (B) geleitet und dem Fischer-Tropsch-Syntheseprozess unterzogen. Im Folgenden wird das CO₂, welches unter anderem durch den Fischer-Tropsch-Syntheseprozess gebildet wurde, in einem Kohlendioxid-Abtrennungsschritt (C) abgetrennt, abgetrenntes CO₂ (d) wird zurückgeleitet in den Erdgas-Reformer (A). Darüber hinaus werden geringe Mengen an CO₂ (b) aus dem Reaktionsprozess abgeführt. Das CO₂-abgereicherte Syntheseprodukt wird einer Olefinaufarbeitung (D) unterzogen, wonach dann die Olefine (O), vorzugsweise leichte Olefine mit C₂-C₈-Ketten, erhältlich sind.

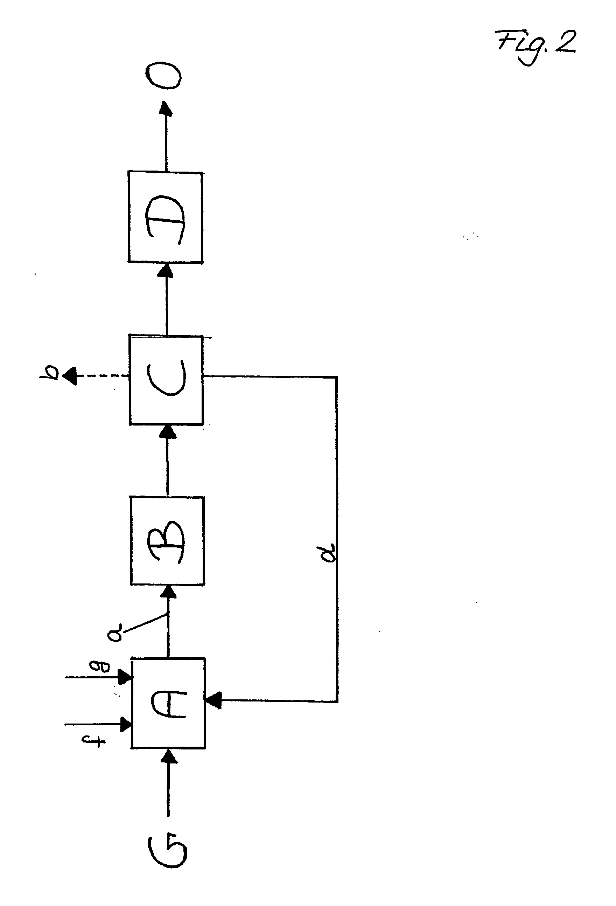

### Figur 2 beschreibt die Erfindung in der Ausgestaltung über den Steam-Reforming Prozess

Das Erdgas (G), im Wesentlichen enthaltend Methan, wird in den Erdgas-Reformer (A) geleitet, wobei zusätzlich Sauerstoff (f) und/oder Wasser (g) zugeführt wird. Durch während des Reaktionsprozesses abgetrenntes Kohlendioxid (d) und Rückführung in den Erdgas-Reformer (A) wird ein Synthesegas mit einem Verhältnis von Wasserstoff zu Kohlenmonoxid von vorzugsweise 1 : 1 eingestellt. Dieses Synthesegas wird dann in den Fischer-Tropsch-Reaktor (B) geleitet und dem Fischer-Tropsch-Syntheseprozess unterzogen. Im Folgenden wird das CO₂, welches unter anderem durch den Fischer-Tropsch-Syntheseprozess gebildet wurde, in einem Kohlendioxid-Abtrennungsschritt (C) abgetrennt und das abgetrennte CO₂ (d) wird zurückgeleitet in den Erdgas-Reformer (A). Darüber hinaus werden geringe Mengen an CO₂ (b) aus dem Reaktionsprozess abgeführt. Das CO₂-abgereicherte Syntheseprodukt wird einer Olefinaufarbeitung (D) unterzogen, durch den dann die Olefine (O), vorzugsweise leichte Olefine mit C₂-C₈-Ketten, erhältlich sind.

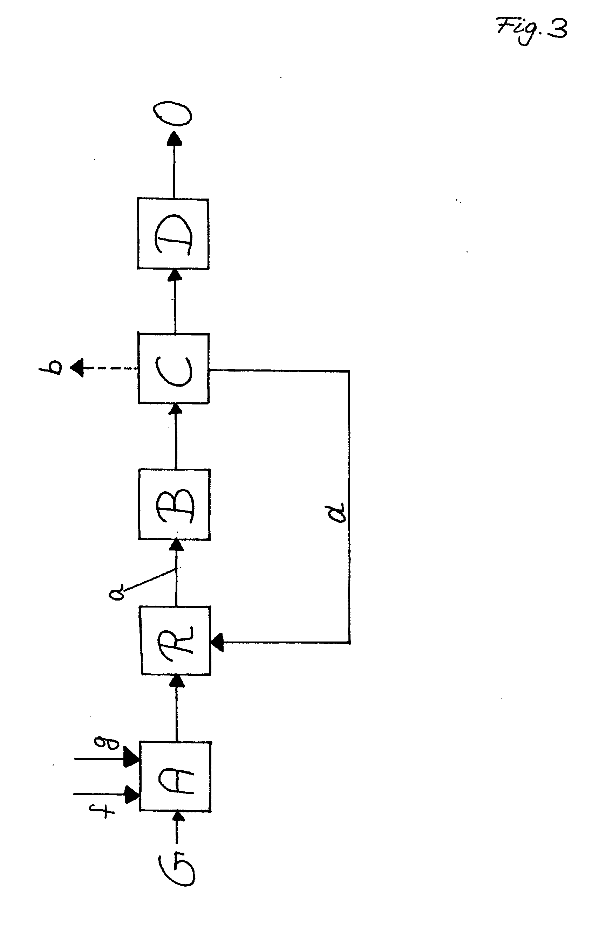

### Figur 3 beschreibt die Erfindung in der Ausgestaltung mit zwischengeschalteten Reverse-Shift-Reaktor

Das Erdgas (G), welches hauptsächlich Methan enthält, wird in den Erdgas-Reformer (A) geleitet. In den Erdgas-Reformer (A) werden Sauerstoff (f) und/oder Wasser (g) geleitet. Aus diesem Erdgas-Reformer (A) wird das Synthesegas in einen Reverse-Shift-Reaktor (R) geleitet. Das während des Prozesses entstehende Kohlendioxid (d) wird in diesen Reverse-Shift-Reaktor (R) geleitet. Es entsteht ein Synthesegas mit einem Verhältnis von Wasserstoff zu Kohlenmonoxid von vorzugsweise 1 : 1 (a). Dieses Synthesegas wird in den Fischer-Tropsch-Reaktor (B) geleitet. Im Folgenden wird das CO₂, welches unter anderem durch den Fischer-Tropsch-Syntheseprozess gebildet wurde, in einem Kohlendioxid-Abtrennungsschritt (C) abgetrennt, das abgetrennte CO₂ (d) wird zurückgeleitet in den Reverse-Shift-Reaktor (R). Darüber hinaus werden geringe Mengen an CO₂ (b) aus dem Reaktionsprozess abgeführt. Das CO₂-abgereichert Syntheseprodukt wird einer Olefinaufarbeitung (D) unterzogen, wonach dann die Olefine (O), vorzugsweise leichte Olefine mit C₂-C₈-Ketten, erhältlich sind.

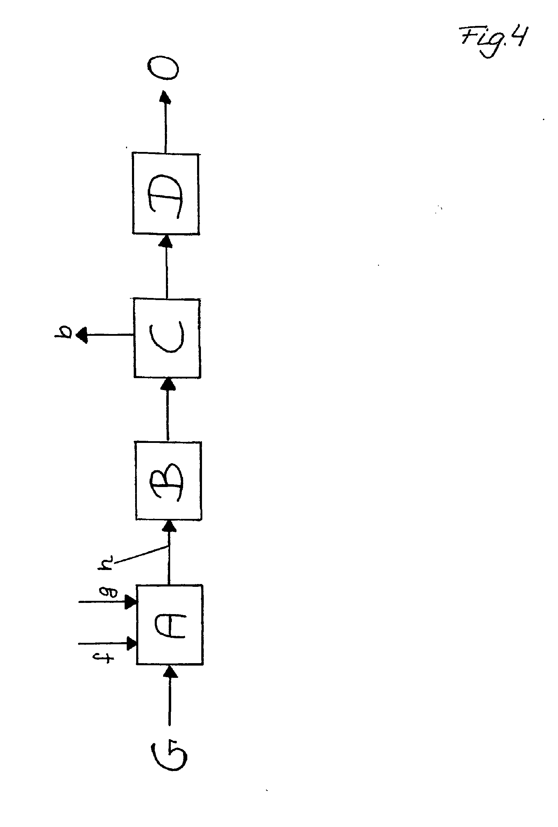

### Figur 4 beschreibt die Herstellung von Olefinen aus Erdgas über Fischer-Tropsch-Reaktion gemäß dem Stand der Technik

Zunächst wird Erdgas (G), welches vorzugsweise Methan enthält, in den Erdgas-Reformer (A) geleitet. Dort hinein werden Sauerstoff (f) und/oder Wasser (g) geleitet, wodurch eine Synthesegasmischung von Wasserstoff zu Kohlenmonoxid im Verhältnis von ungefähr 2 : 1 (h) erhalten wird. Diese Synthesegasmischung wird in den FischerTropsch-Reaktor (B) geleitet, das resultierende Produkt einem Kohlendioxid-Abtrennungsschritt (C) unterzogen und das bei der Reaktion freiwerdende Kohlendioxid aus dem Syntheseprozess entfernt. Das zurückbleibende Rohprodukt wird einer Olefinaufarbeitung (D) unterzogen und daraus dann die Olefine (O) erhalten.

### Beispiele

### Beispiel 1

Für die Fischer-Tropsch-Synthese wurde ein Carbonyl-Eisenpulver-Katalysator mit spherischen Primärpartikeln analog zu WO 2009/013174 hergestellt. Die Reaktion wurde in einer Standard Fischer-Tropsch-Synthese-Apparatur durchgeführt. Die Reaktionstemperatur betrug 340 °C bei 25 bar Druck. Es wurde eine Synthesegasmischung im Verhältnis von Wasserstoff zu Kohlenmonoxid von 1 : 1 eingestellt. Die Produktzusammensetzung wurde in zeitgleichen Abständen über einen Zeitraum von mehreren Stunden insgesamt 6 mal analysiert. Die Durchschnittswerte sind im Folgenden in [Mol-%] angegeben:
Kohlenmonoxid-Umsatz: 98,6 %
Anteil der C₂-C₄-Fragmente: 33 %
davon Olefine: 25,5 %
davon gesättigte Kohlenwasserstoffe: 7,5 %
Anteil an Methangas: 13 %.

### Beispiel 2

Die Versuchsreihe wurde analog zu Beispiel 1 durchgeführt, wobei das Verhältnis im Synthesegas von Wasserstoff zu Kohlenmonoxid 0,9 : 1 betrug. Die Produktzusammensetzung wurde in zeitgleichen Abständen über einen Zeitraum von mehreren Stunden insgesamt 5 mal analysiert. Die Durchschnittswerte sind im Folgenden in [Mol-%] angegeben:
Kohlenmonoxid-Umsatz: 97,0 %
Anteil der C₂-C₄-Fragmente: 54 %
davon Olefine: 47 %
davon gesättigte Kohlenwasserstoffe: 7 %
Anteil an Methangas: 10 %.

### Beispiel 3 (Vergleichsbeispiel)

Der Prozess wurde analog zu den Reaktionsbedingungen aus Beispiel 1 gefahren, wobei die Synthesegaszusammenmischung von Wasserstoff zu Kohlenmonoxid im Verhältnis 2 : 1 eingestellt wurde. Die Produktzusammensetzung wurde in zeitgleichen Abständen über einen Zeitraum von mehreren Stunden insgesamt 6 mal analysiert. Die Durchschnittswerte sind im Folgenden in [Mol-%] angegeben:
Kohlenmonoxid-Umsatz: 98,1 %
Anteil der C₂-C₄-Fragmente: 26,5 %
davon Olefine: 19 %
davon gesättigte Kohlenwasserstoffe: 7,5 %
Anteil an Methangas: 16 %.

Wie aus den Beispielen 1 und 2 und dem Vergleichsbeispiel (Beispiel 3) ersichtlich wird, kann sowohl die Kohlenstoffausbeute insgesamt (Mol-%) sowie die Selektivität von C₂-C₄-Olefinen gegenüber aliphatischen, gesättigten C₂-C₄-Kohlenwasserstoffen durch das Verhältnis von Wasserstoff zu Kohlenmonoxid in der Synthesegasmischung gesteuert werden.

## Patentansprüche

1. Verfahren zur Herstellung von Olefinen umfassend die folgenden Schritte:
a) Herstellung eines Kohlenmonoxid und Wasserstoff enthaltenden Synthesegases, wobei das Synthesegas aus einem Gemisch enthaltend Kohlenwasserstoffe mit einem Verhältnis von Wasserstoff zu Kohlenstoff von 0,5 bis 4: 1 hergestellt wird.
b) Einspeisung von aus Schritt d) rückgeleitetem Kohlendioxid in das Synthesegas während oder im Anschluss an die Synthesegasherstellung gemäß Schritt a),
c) Umsetzen des in Schritt b) erhaltenen Synthesegases mit einem Wasserstoff-zu-Kohlenmonoxid-Verhältnis von ≤ 1,2 : 1 zu Olefinen in Gegenwart eines Fischer-Tropsch-Katalysators,
d) Abtrennen des im Reaktionsprodukt von Schritt c) enthaltenen Kohlendioxids,
wobei das Verhältnis von Wasserstoff zu Kohlenmonoxid in Schritt c) durch Schritt b) eingestellt wird.

2. Verfahren gemäß Anspruch 1, **dadurch gekennzeichnet, dass** der Fischer-Tropsch-Katalysator in Schritt c) ein eisen- oder kobalthaftiger Heterogenkatalysator ist.

3. Verfahren gemäß Anspruch 1 oder 2, **dadurch gekennzeichnet, dass** der Fischer-Tropsch-Katalysator in Schritt c) ein Carbonyl-Eisenpulver-Katalysator mit spherischen Primärpartikeln ist.

4. Verfahren gemäß einem der Ansprüche 1 bis 3, **dadurch gekennzeichnet, dass** C₂-C₈-Olefine hergestellt werden.

5. Verfahren gemäß einem der Ansprüche 1 bis 4, **dadurch gekennzeichnet, dass** C₂-C₄-Olefine hergestellt werden.

6. Verfahren gemäß einem der Ansprüche 1 bis 5, **dadurch gekennzeichnet, dass** das Synthesegas in Schritt a) aus Methan und/oder Erdgas hergestellt wird.

7. Verfahren gemäß einem der Ansprüche 1 bis 6, **dadurch gekennzeichnet, dass** das Synthesegas in Schritt a) hergestellt wird über einen Prozess ausgewählt aus der Gruppe Steam-Reforming, autothermales Reforming, nicht-katalytische partelle Oxidation, katalytische partielle Oxidation, Wärmeaustausch-Reforming, Kompakt-Reforming und Dry-Reforming.

8. Verfahren gemäß einem der Ansprüche 1 bis 7, **dadurch gekennzeichnet, dass** das Synthesegas in Schritt a) über einen Steam-Reforming oder Dry-Reforming-Prozess hergestellt wird.

9. Verfahren gemäß einem der Ansprüche 1 bis 8, **dadurch gekennzeichnet, dass** Wasserstoff zu Kohlenmonoxid in Schritt c) in einem Verhältnis von 1 ,2 : 1 bis 1 : 1,2 zueinander vorliegen.

10. Verfahren gemäß einem der Ansprüche 1 bis 9, **dadurch gekennzeichnet, dass** Wasserstoff zu Kohlenmonoxid in Schritt c) im Verhältnis 1,1 : 1 bis 1 : 1,1 zueinander vorliegen.

11. Verfahren gemäß einem der Ansprüche 1 bis 10, **dadurch gekennzeichnet, dass** Wasserstoff zu Kohlenmonoxid in Schritt c) im Verhältnis 1 : 1,1 zueinander vorliegen.

12. Verfahren gemäß einem der Ansprüche 1 bis 11, **dadurch gekennzeichnet, dass** die Prozesstemperatur in Schritt c) 100 °C bis 500 °C beträgt.

13. Verfahren gemäß einem der Ansprüche 1 bis 11, **dadurch gekennzeichnet, dass** der Druck in Schritt c) zwischen 10 und 50 bar beträgt.

## Claims

1. A process for preparing olefins, which comprises the following steps:
a) preparation of a synthesis gas comprising carbon monoxide and hydrogen, where the synthesis gas is prepared from a mixture comprising hydrocarbons having a ratio of hydrogen to carbon of 0.5-4:1,
b) introduction of carbon dioxide recirculated from step d) into the synthesis gas during or after the preparation of synthesis gas as per step a),
c) conversion of the synthesis gas having a hydrogen to carbon monoxide ratio of ≤ 1.2:1 which is obtained in step b) into olefins in the presence of a Fischer-Tropsch catalyst,
d) removal of the carbon dioxide comprised in the reaction product from step c),
where the ratio of hydrogen to carbon monoxide in step c) is set via step b).

2. The process according to claim 1, wherein the Fischer-Tropsch catalyst in step c) is an iron- or cobalt-comprising heterogeneous catalyst.

3. The process according to claim 1 or 2, wherein the Fischer-Tropsch catalyst in step c) is a carbonyl iron powder catalyst having spherical primary particles.

4. The process according to any of claims 1 to 3, wherein C₂-C₈-olefins are prepared.

5. The process according to any of claims 1 to 4, wherein C₂-C₄-olefins are prepared.

6. The process according to any of claims 1 to 5, wherein the synthesis gas is prepared from methane and/or natural gas in step a).

7. The process according to any of claims 1 to 6, wherein the synthesis gas is prepared by a process selected from the group consisting of steam reforming, autothermal reforming, non-catalytic partial oxidation, catalytic partial oxidation, heat exchange reforming, compact reforming and dry reforming in step a).

8. The process according to any of claims 1 to 7, wherein the synthesis gas is prepared via a steam reforming or dry reforming process in step a).

9. The process according to any of claims 1 to 8, wherein the hydrogen to carbon monoxide ratio in step c) is from 1.2:1 to 1:1.2.

10. The process according to any of claims 1 to 9, wherein the hydrogen to carbon monoxide ratio in step c) is from 1.1:1 to 1:1.1.

11. The process according to any of claims 1 to 10, wherein the hydrogen to carbon monoxide ratio in step c) is 1:1.1.

12. The process according to any of claims 1 to 11, wherein the process temperature in step c) is from 100°C to 500°C.

13. The process according to any of claims 1 to 11, wherein the pressure in step c) is in the range from 10 to 50 bar.

## Revendications

1. Procédé de fabrication d'oléfines comprenant les étapes suivantes :
a) la fabrication d'un gaz de synthèse contenant du monoxyde de carbone et de l'hydrogène, le gaz de synthèse étant fabriqué à partir d'un mélange contenant des hydrocarbures ayant un rapport entre l'hydrogène et le carbone de 0,5 à 4:1 ;
b) l'introduction du dioxyde de carbone recyclé de l'étape d) dans le gaz de synthèse pendant ou après la fabrication du gaz de synthèse selon l'étape a) ;
c) la mise en réaction du gaz de synthèse obtenu à l'étape b) ayant un rapport hydrogène sur monoxyde de carbone de ≤ 1,2:1 pour former des oléfines en présence d'un catalyseur de Fischer-Tropsch ;
d) la séparation du dioxyde de carbone contenu dans le produit de réaction de l'étape c) ;
dans lequel le rapport entre l'hydrogène et le monoxyde de carbone à l'étape c) est ajusté par l'étape b).

2. Procédé selon la revendication 1, **caractérisé en ce que** le catalyseur de Fischer-Tropsch à l'étape c) est un catalyseur hétérogène contenant du fer ou du cobalt.

3. Procédé selon la revendication 1 ou 2, **caractérisé en ce que** le catalyseur de Fischer-Tropsch à l'étape c) est un catalyseur à base de poudre de fer-carbonyle contenant des particules primaires sphériques.

4. Procédé selon l'une quelconque des revendications 1 à 3, **caractérisé en ce que** des oléfines en C₂-C₈ sont fabriquées.

5. Procédé selon l'une quelconque des revendications 1 à 4, **caractérisé en ce que** des oléfines en C₂-C₄ sont fabriquées.

6. Procédé selon l'une quelconque des revendications 1 à 5, **caractérisé en ce que** le gaz de synthèse à l'étape a) est fabriqué à partir de méthane et/ou de gaz naturel.

7. Procédé selon l'une quelconque des revendications 1 à 6, **caractérisé en ce que** le gaz de synthèse à l'étape a) est fabriqué par un procédé choisi dans le groupe constitué par le reformage à la vapeur, le reformage autothermal, l'oxydation partielle non catalytique, l'oxydation partielle catalytique, le reformage avec échange de chaleur, le reformage compact et le reformage à sec.

8. Procédé selon l'une quelconque des revendications 1 à 7, **caractérisé en ce que** le gaz de synthèse à l'étape a) est fabriqué par un procédé de reformage à la vapeur ou de reformage à sec.

9. Procédé selon l'une quelconque des revendications 1 à 8, **caractérisé en ce que** l'hydrogène et le monoxyde de carbone sont présents à l'étape c) en un rapport de 1,2:1 à 1:1,2 l'un par rapport à l'autre.

10. Procédé selon l'une quelconque des revendications 1 à 9, **caractérisé en ce que** l'hydrogène et le monoxyde de carbone sont présents à l'étape c) en un rapport de 1,1:1 à 1:1,1 l'un par rapport à l'autre.

11. Procédé selon l'une quelconque des revendications 1 à 10, **caractérisé en ce que** l'hydrogène et le monoxyde de carbone sont présents à l'étape c) en un rapport de 1:1,1 l'un par rapport à l'autre.

12. Procédé selon l'une quelconque des revendications 1 à 11, **caractérisé en ce que** la température de procédé à l'étape c) est de 100 °C à 500 °C.

13. Procédé selon l'une quelconque des revendications 1 à 11, **caractérisé en ce que** la pression à l'étape c) est comprise entre 10 et 50 bar.
